# EUROPEAN PATENT APPLICATION

(11) **EP 3 207 982 A1**
(43) Date of publication of application: **23.08.2017**
(21) Application number: 16156146.9
(22) Date of filing: 17.02.2016
(51) Int. Cl.: B01J 19/00, B01L 3/00, C12Q 1/68, B01L 3/02

(54) **IMPROVED DROPLET SEQUENCING APPARATUS AND METHOD**

(71) Applicant: Base4 Innovation Limited, Cambridge CB3 0FA (GB)
(72) Inventor: The designation of the inventor has not yet been filed
(74) Representative: Briscoe, Paul Brian

(57) **Abstract**

A first apparatus for sequencing a polynucleotide analyte is provided and comprises;
• a first zone in which a stream of single nucleotide phosphates is generated by progressive pyrophosphorolysis of a molecule of the analyte attached to a particle and exposed to a flowing aqueous medium;
• a second zone in which a corresponding stream of aqueous droplets is generated from the aqueous medium and the nucleotide stream and wherein at least some of the droplets contain a single nucleotide triphosphate and
• a third zone in which each droplet is stored and optionally interrogated to reveal a property characteristic of the single nucleotide it may contain.

It is characterised in that the first zone includes at least one chemically-modified substrate adapted to bind temporarily to a particle having at least two different regions adapted to bind respectively to the substrate and to the analyte.

Suitably the particles comprise at least one metal coated first region(s) and at least one second region(s) comprised of reactive sites to which the analyte can be attached. In one embodiment the two regions each comprise hemispheres of a substantially spherical bead.

## Description

This invention relates to an improved apparatus and method for sequencing polynucleotides such as DNA fragments derived from genetic material of natural origin.

Next generation sequencing of genetic material is already making a significant impact on the biological sciences in general and medicine in particular as the unit cost of sequencing falls in line with the coming to market of faster and faster sequencing machines. In our previous applications WO 2014/053853 and WO 2014/053854 we have described a new sequencing method and associated apparatus which involves progressive digestion of a polynucleotide analyte to generate a stream of single-nucleobase nucleotides (hereinafter 'single nucleotides'), preferably an ordered stream thereof generated by pyrophosphorolysis, each of which can be captured one-by-one into corresponding microdroplets. Thereafter, each droplet can be chemically and/or enzymatically manipulated to reveal the particular single nucleotide it originally contained. In one embodiment, these chemical and/or enzymatic manipulations comprise a method involving the use of one or more multi-component oligonucleotide probe types each of which is adapted to be able to selectively capture one of the single nucleotide types from which the analyte is constituted; for example in the case of naturally occurring DNA one of the four types corresponding to the nucleobases cytosine, thymine, guanine and adenine. Typically, in each such probe type, one of the oligonucleotide components comprises distinct and characteristic fluorophores and in the probe's unused state the ability of these fluorophores to fluoresce remains extinguished by virtue of either the presence of quenchers located close-by or by self-quenching. Thereafter, once a particular probe has captured its corresponding single nucleotide, it becomes susceptible to exonucleolysis thereby causing the fluorophores to become separated from the quenchers and/or each other and thus enabling them to fluoresce freely at their characteristic wavelength. By this means, the nature of the single nucleotide originally present in each droplet can be inferred from spectroscopic analysis.

We have now developed an improved method for use with sequencing methods of this type which allows the initial polynucleotide digestion step to be easily and reliably conducted and in a way which can be multiplexed to work with many thousands of droplet generation sites (for example the multiple nozzles of a printer). The improvement involves attaching the polynucleotide to be analysed (hereinafter the analyte) on the surface of a particle including at least two different surface regions and thereafter temporarily immobilising the particle at a location within the apparatus where digestion is to occur. We are aware that US6471917 has previously described systems and techniques for assembling small solid beads into an organized array but to the best of our knowledge such technologies have not been applied to holding single analyte molecules under flow conditions or to single molecule sequencing applications.

Thus, according to a first aspect of the invention there is provided an apparatus for sequencing a polynucleotide analyte comprising;
- a first zone in which a stream of single nucleotides is generated by progressive pyrophosphorolysis of a molecule of the analyte attached to a particle and exposed to a flowing aqueous medium;
- a second zone in which a corresponding stream of aqueous droplets is generated from the aqueous medium and the nucleotide stream and wherein at least some of the droplets contain a single nucleotide and
- a third zone in which each droplet is stored and/or interrogated to reveal a property characteristic of the single nucleotide it may contain;
characterised in that the first zone includes at least one chemically-modified substrate adapted to bind temporarily to a particle having at least two different regions adapted to bind respectively to the substrate and to the analyte.

The polynucleotide to be sequenced in the apparatus of the invention is suitably a double-stranded nucleic acid and may have a nucleotide chain length which can in principle be unlimited; for example up to and including the many millions of nucleotide base pairs found in a fragment of a genome. In one embodiment, the analyte will therefore be at least 50, preferably at least 150 nucleotide base pairs long; suitably it will be greater than 500, greater than 1000 and in some cases 5000+ nucleotide base pairs long. In one embodiment, the analyte is DNA of natural origin (e.g. genetic material derived from a plant, animal, bacterium or a virus) although the method is equally applicable to the sequencing of partially or wholly synthetic DNA or indeed other nucleic acids made up wholly or in part of nucleotides comprised of nucleotide bases that are not commonly encountered in nature; i.e. nucleotides having nucleobases other than adenine, thymine, guanine, cytosine and uracil. Examples of such nucleobases include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentenyladenosine, uridine-5-oxyacetic acid-methylester, uridine-5-oxyacetic acid, wybutoxosine, wybutosine, pseudouridine, queuosine, 2-thiocytidine, 5-methyl-2-thiouridine, 2-thiouridine, 4-thiouridine, 5-methyluridine, 2-O-methyl-5-methyluridine and 2-O-methyluridine.

In the first zone of the apparatus, the analyte is progressively pyrophosphorolysed in the 3-5' direction to generate a stream of single nucleotide triphosphates the order of which corresponds to that of the sequence of the analyte. The pyrophosphorolysis itself is generally carried out at a temperature in the range 20 to 90°C in the presence of a reaction medium including an enzyme such as a polymerase. Suitably, it is carried out so that the single nucleotides are continually removed from the region of pyrophosphorolysis around the particle by a flowing aqueous medium. In another embodiment, this medium is buffered and also contains those other components needed to sustain the pyrophosphorolysis reaction (polymerase, pyrophosphate anion, magnesium cation etc.). In yet another embodiment, the medium additionally contains one or more of (a) the probe types specified below (or probes having an equivalent function); (b) the various chemicals and enzymes required to cause the probe to bind to and capture the relevant single nucleotide (e.g. polymerase and/or ligase) and (3) an enzyme required to cause subsequent exonucleolysis of the used probe to occur. In one embodiment, some or all of these components are introduced together or in stages into the flowing aqueous medium or the droplets formed therefrom (as the case may be) at some other point(s) in the first, second or third zones. For example, subsequent introduction of some or all of these components directly into the droplets can be achieved by injection using an injector or by droplet coalescence.

Preferably the apparatus is designed to be operable so that the rate of pyrophosphorolysis is as fast as possible and in one embodiment this rate lies in the range from 1 to 50 single nucleotides per second. Further information about the pyrophosphorolysis reaction as applied to the progressive degradation of polynucleotides can be found for example in J. Biol. Chem. 244 (1969) pp. 3019-3028.

The first zone of the apparatus is characterised by including a substrate which is adapted to bind temporarily to a particle having two different surface regions. Suitably this substrate comprises a surface located within the first zone which is modified to be releasably attachable to a corresponding particle bearing a molecule of the analyte; for example via a chemically-modified metal coating. In one embodiment, this particle comprises a bead; for example a microbead, made of an inert material such as glass, silica, alumina, a metal or a non-degradable polymer. In another embodiment, the particle has a core of magnetic material enabling it to be manipulated magnetically.

The two different surface regions on the particle are adapted to bind to complementary sites on the substrate and the analyte respectively. In one embodiment, the first of these regions is comprised of a chemically-modified metal coating; for example, a functionalised coating of gold, silver, copper or other metal. In another embodiment, the second of these regions is comprised of one or more reactive sites on the particle specifically adapted to bind physically or chemically to a molecule of the analyte.

There are many ways in which these reactive sites on the particle and substrate can be created. For example in the case of the first region on the particle, it can be prepared by first partially coating the particles with metal using a known method such as metal vapour deposition, atomic layer deposition, plasma deposition and the like. Thereafter, the metal surface can be chemically modified to introduce one or more first moieties which can reversibly bind to complementary second moieties on the substrate. In one embodiment, these pairs of first and second moieties are chosen so that the bond created between them can be reversibly made and broken at ambient or near ambient temperatures. By this means the bead can be attached to the substrate, the analyte digested by pyrophosphorolysis and thereafter the bead detached from the substrate enabling the latter to then receive another fresh bead. In one embodiment, these pairs of first and second moieties can lead to the formation of protein complexes stable under the conditions of pyrophosphorolysis e.g. by using avidin or streptavidin moieties with complementary biotin moieties. In another, these pairs can create a labile chemical bond; as for example in a polyhistidine/chelated metal ion pair (bond broken at low pH or in the presence of imidazole or strong metal chelator); a boronic acid/suitable carbohydrate pair (bond broken at low pH) or a maleimido/selenol pair (bond broken using meta-chloroperoxybenzoic acid). In these cases, the particle can be subsequently detached by modifying the pH or composition of the aqueous medium flowing through the first zone. Thus in one embodiment of the apparatus the first zone further includes means for introducing and removing the particle from the first zone or means for introducing and removing a particle-detaching means thereinto.

In one preferred embodiment, the pair of moieties mentioned above comprises a polyhistidine moiety comprised of a plurality of histidine residues (preferably greater than six) and a chelator moiety selected from for example a nitrotriacetate (NTA) or iminodiacetate (IDA) salt derivative of a transition metal such as cobalt, copper or nickel. It will be appreciated that these first and second moieties can be deployed on the particle and substrate respectively either way around.

Likewise, in the case of the particle's second region there are many ways in which the analyte-reactive sites can be created. For example, in one embodiment an uncoated surface of the particle may be primed with a functionalising agent, for example in the case of a silica, alumina or glass bead, an epoxysilane, an aminohydrocarbylsilane or a mercaptosilane, to create chemically reactive sites to which the analyte can be attached. Thereafter and in use these reactive sites can be treated with a derivative of the analyte which has been correspondingly modified to include a terminal primer-reactive group; for example in one embodiment an amine, succinyl or thiol group. In another embodiment, chemical attachment can take place via ligation to adaptor oligonucleotides or via the types of protein complexes mentioned above.

In yet another embodiment, the particle so primed will comprise a second region having only one analyte-reactive site so that only one molecule of the analyte can be attached. This can be important if the analyte is a small polynucleotide fragment since otherwise multiple molecules tend to become attached during preparation which is undesirable. It is of a lesser concern if the polynucleotide fragment is large since steric effects will work to militate against such an outcome. Suitably, the particle will have a maximum diameter in the range 0.5 to 5 microns.

In one preferred embodiment, the particles are spherical beads and the first and second regions comprise hemispherical regions abutting one another on the surface thereof to produce what may be referred to as a 'Janus' bead. In this embodiment the particle is suitably a bead having a magnetic core; for example those sold under the name Dynabeads^{®}.

The first zone is suitably a chamber of microfluidic dimensions containing a structure including the substrate or in another embodiment comprises a microfluidic tube whose internal wall has in part been coated with the substrate. In one embodiment the substrate may further include a seating into which the particle can be close-fitted. In another embodiment, the particle may be held in place at the desired location by surface binding or magnetically. In yet another embodiment the particles may have, for example, a ferromagnetic core or surface enabling them to be guided to the binding or seating region by application of an electromagnet in close proximity thereto.

After pyrophosphorolysis, the aqueous medium issuing forth from the first zone contains the single nucleotides separated spatially and temporally from each other and arranged in an order corresponding to that of the nucleotide sequence of the analyte. Thereafter, in the second zone this aqueous medium is then converted into a corresponding stream of aqueous droplets, suitably microdroplets, at least some of which contain a single nucleotide. In one embodiment, this is achieved by causing the aqueous medium to issue forth from a droplet-generating head of suitable dimensions and geometry into a flowing carrier medium comprising an immiscible liquid such as a mineral or hydrocarbon oil; for example silicone oil. Thereafter, the droplet stream can be microfluidically manipulated and delivered to the third zone for storage and interrogation. In a more preferred embodiment however, the second zone includes at least one printer nozzle which dispenses the aqueous stream in the form of droplets directly onto a third zone comprising an array of droplet-receiving structures arranged on a sheet coated with the immiscible solvent. In one embodiment these structures are wells whose capacities are greater than the volume of the dispensed droplet thereby enabling further aqueous droplets containing other reactants to be introduced either subsequently or beforehand. In another, the printer nozzle(s) and sheet are moveable relative to one another. In yet another embodiment the immiscible liquid comprises a uv-curable monomer to enable the droplets to be finally encased in a transparent solid polymer matrix.

To avoid the risk that a given microdroplet contains more than one single nucleotide, it is preferred to release the single nucleotides in the pyrophosphorolysis step and/or to adjust the flow of the aqueous medium through the first zone so that each filled microdroplet generated in the second zone is separated on average by from 1 to 20 preferably 2 to 10 empty ones. Suitably the droplets employed are microdroplets have a diameter less than 100 microns, preferably less than 50 microns, more preferably less than 20 microns and even more preferably less than 15 microns. Most preferably of all their diameters are in the range 2 to 20 microns. In one embodiment, the microdroplet flow rate from the second to third zones is in the range 50 to 3000 droplets per second preferably 100 to 2000.

In the third zone each stored droplet is interrogated to reveal a property characteristic of the single nucleotide that it contains. Although this interrogation can be made separate from the apparatus, for example optionally within another specialised stand-alone device such as a spectrometer, it is preferably carried out using an interrogation means which is an integral part of the third zone. In one preferred embodiment the interrogation means comprises a laser and a photodetector to detect fluorescence or Raman-scattered radiation and generate a data stream characteristic of the sequence of the analyte. In another the interrogation means further comprises a microprocessor integral therewith for analysing the data stream. Alternatively this duty may be performed by a stand-alone PC or the like.

The property to be detected by the interrogation means can in principle be any characteristic property of the single nucleotide which is made manifest directly or indirectly. However, in one preferred embodiment the second and third zones are used to create and interrogate droplets in which the single nucleotides they contain have been previously captured selectively by probes which are capable of fluorescing substantially only after they have been used and undergone subsequent exonucleolysis. In general terms, this is achieved by ensuring that the apparatus is capable of processing droplets which at some point contain a first component comprising molecules of at least one probe type selective for one of the various single nucleotides from which the analyte is constructed and enzymes enabling incorporation of the single nucleotide into its corresponding probe; using e.g. polymerase and/or ligase. Each droplet will also suitably contain, at some point, further components comprised of some or all of; a 3'-5' exonuclease or a polymerase demonstrating an equivalent activity; a pyrophosphatase, buffer, surfactants and other components conventional in the biochemical art. In one embodiment, some, all or any permutation of these probes, enzymes, exonuclease and other components are introduced into the original aqueous medium in the first zone or contained within the original aqueous medium feed itself. In another some, all or any permutation of these probes, enzymes, exonuclease and other components are introduced into the aqueous medium as or after each droplet is created. In this case, these materials may be introduced into the droplet by for example direct injection or by locating them first in secondary droplets comprising a secondary droplet stream and thereafter merging droplets from that stream one-by-one with corresponding droplets in the primary stream.

In one preferred embodiment the droplets containing the single nucleotide are printed by the printer nozzle into wells on a plate or sheet as described above and the first and other components printed into these wells by means of other printer nozzles as for example described in our co-pending European patent application 15002007.1.

As regards the probes themselves, one class which may advantageously be used is that based on the various capture systems and their modes of utilisation taught in our patent applications WO 2014/053853, WO 2014/053854, WO 2014/167323 and WO 2014/167324 to which the reader is directed for further information to be regarded as included herein. Briefly, one sub-class of these probes, in their unused state, is characterised by comprising a single-stranded nucleotide region the ends of which are each attached to two different double-stranded oligonucleotide regions. Preferably, at least one of the oligonucleotide regions comprises multiple fluorophores exhibiting characteristic fluorescence and optionally quenchers. The fluorophores are arranged on the oligonucleotide region so that that the fluorescence they exhibit is substantially less than when the same number of fluorophores is bound to a corresponding number of single nucleotides. Preferably the probes in their unused state exhibit either no or substantially no fluorescence at all. These probes work by capturing their corresponding single nucleotide to generate a used probe which is then susceptible to exonucleolysis leading to the generation of multiple single nucleotides bearing the fluorophores which are then able to fluoresce fully. In another preferred sub-class, these probes are composed of two components; complementary i- and j-shaped oligonucleotides, either or both of which can be labelled, which can be hybridised together in the presence of their target single nucleotide into a substantially double-stranded oligonucleotide which can also be digested by an exonuclease as explained above.

In yet another preferred sub-class, disclosed in our pending application WO 2016/012789 and to which the reader is also directed for further information, the probe comprises (a) a first single-stranded oligonucleotide labelled with characteristic fluorophores in an undetectable state and (b) second and third single-stranded oligonucleotides capable of hybridising to complementary regions on the first oligonucleotide. In the case of this particular sub-class, the used probe can be digested with an enzyme having double-stranded exonucleolytic activity to yield the detectable elements in a detectable state and a single-stranded fourth oligonucleotide which is at least in part the sequence complement of the first oligonucleotide. As a consequence, the fourth oligonucleotide can be caused to react with another labelled first oligonucleotide to produce a substantially double-stranded oligonucleotide product again corresponding to the used probe. By repeating both this and the exonucleolysis step cyclically, the number of free fluorophores capable of fluorescing can be caused to cascade and increase significantly. Most preferred items in the class comprise those oligonucleotides in which the second and third oligonucleotides comprise a single oligonucleotide so that the fourth oligonucleotide when created by addition of the target single nucleotide is a closed single-stranded loop.

In a second aspect of the invention there is provided a method of pyrophosphorolysing a polynucleotide comprising the steps of (1) binding the polynucleotide to a particle the surface of which is adapted to (a) bind the polynucleotide and (b) bind a substrate surface reversibly; (2) binding the particle to the substrate surface; (3) performing pyrophosphorolysis on the polynucleotide bound to the particle in a flowing medium and (4) releasing the particle from the surface.

In a third aspect of the invention there is provided an apparatus for sequencing a polynucleotide analyte characterised by comprising:
- at least one microfluidic chamber containing a substrate chemically-modified to bind to a complementary particle bearing a molecule of the analyte;
- a means for delivering and retrieving the particle to and from the chamber;
- microfluidic channels for passing an aqueous pyrophosphorolysing medium through the microfluidic chamber and across the substrate;
- at least one first printer nozzle adapted to receive a nucleotide-containing aqueous medium from the microfluidic chamber and to create microdroplets therefrom;
- at least one sheet moveable relative to the first printer nozzle and patterned with an array of wells for receiving the nucleotide-containing microdroplets from the printer nozzle(s);
- at least one additional printer nozzle type moveable relative to the sheet each additional type being adapted to deliver a microdroplet containing a different reagent into the wells either before or after a nucleotide-containing microdroplet has been received;
- a means for moving the first and additional printer nozzle types relative to the sheet and
- a means for coating the sheet with a liquid immiscible with the nucleotide-containing medium.

In one embodiment this apparatus further comprises at least one laser for interrogating the contents of the wells and at least one photodetector for detecting electromagnetic radiation, preferably fluorescence, originating from the wells each being moveable relative to the sheet. In another embodiment the apparatus comprises a microprocessor for analysing a data stream from the photodetector. In yet another embodiment the apparatus includes a UV source for curing the immiscible liquid where the latter is a uv-curable monomer.

An apparatus according to the invention is now illustrated by the following Figure which schematically illustrates a sequencing device according to the present invention in which aqueous droplets containing a single nucleotide and nucleotide-detecting oligonucleotide probe system are created, printed onto a sheet and interrogated.

A ten micron diameter microfluidic printing nozzle 1 is connected to a microfluidic chamber 2 containing a gold coated glass plate 3 to which are attached poly(L-histidine) moieties (see below for preparation). Prior to use 1, 2 and 3 are cleaned and treated with an anti-biofouling agent. A Dynabeads^{®} His-Tag Isolation and Pulldown bead (ex ThermoFisher Scientific) 4 bearing a polynucleotide analyte in a liquid carrier is introduced into 2 by preliminary up-suction through 1 and attached to 3 via the bead's Cobalt NTA tags so that the part of its outer surface bearing the analyte protrudes into 2. After the liquid carrier has been removed by the flushing of 2, a stream of reaction medium 5 is caused to flow through 2 from upstream point of introduction 6. 5 comprises an aqueous buffer (pH 7.5) at 37°C, containing Bst Large Fragment DNA Polymerase and a 2 millimoles per litre solution of each of sodium pyrophosphate and magnesium chloride. The temperature of 4 is maintained at 37°C and under these conditions the polymerase progressively digests the analyte in the 3' to 5' direction thereby releasing a stream of single nucleotides (deoxyribonucleotide triphosphates) which are then carried out of 1 as droplets 7 by the flow of 5. The order of single nucleotides in the exiting stream of 7 thus corresponds to the original nucleotide sequence of the analyte. Droplets 7 are in turn printed into corresponding droplet-receiving locations 8 (in one embodiment, wells) arranged in an array on a plastic sheet 9 and mounted on assembly 10 which is moveable in the plane of 9. 9 is covered with a film of silicone oil 11. Aqueous droplets 12 containing pyrophosphatase are then introduced into each used location followed by, after intervening periods of incubation, droplets 13 containing a probe system according to WO 2016/012789, a ligase and a polymerase and then droplets 14 containing an exonuclease from printer nozzle assemblies 15, 16 and 17 respectively and movement of 9 relative to them. In one embodiment one of 16 and 17 may be omitted and droplets containing both the probe and exonuclease delivered in one go.

After these additions are complete and incubation has occurred, the contents of each location 8 is then interrogated by a detection system comprising laser 18 and corresponding photodetector 19 tuned to the appropriate wavelengths for the excitation and detection of fluorescence from the fluorescent dyes used in the probe system. The detection of significant fluorescence at a given wavelength above a pre-determined threshold then indicates the presence of a single nucleotide base of a given type within the droplet. Thus as 9 is moved relative to 18 and 19 each location 8 is interrogated in turn so that the sequence of nucleotide bases in the original polynucleotide analyte can in effect be read off as a data stream to be subsequently analysed by microprocessor (not shown).

### Example 1- Preparation of a functionalised substrate.

A clean, glass plate previously coated with gold by atomic layer deposition was immersed in a mixture of sulphuric acid and hydrogen peroxide at 80°C for one hour. Thereafter it was washed with distilled water and then dried under a stream of nitrogen gas. The dried plate was then treated with an ethanol solution of HS-(CH₂)₁₁-EG₆-NH₂.HCl in an Eppendorf tube for 24-72 hours at room temperature in darkness. The treated plate was then washed with ethanol and again dried under nitrogen gas.

Poly(L-histidine) was dissolved in a 1:1 mixture of DMF and a saline solution containing 2-(N-morpholino)ethanesulfonic acid (pH5) followed by the addition of (N-hydroxysulfosuccinimide) and 1-ethyl-3-(3-dimethylaminopropyl)carbodiimide. The functionalised gold plate was then added and the reaction allowed to proceed for 24 hours. At the end of this time the plate was washed and dried under nitrogen.

### Example 2 - Preparation and use of a typical probe system.

An example of an oligonucleotide probe system that may be used in the system described above is now described in detail.

A single-stranded first oligonucleotide A is prepared, having the following nucleotide sequence:
5'TCGTGCCTCATCGAACATGACGAGGXXQXXGGTTTGTGGT3'
wherein A, C, G, and T represent nucleotides bearing the relevant characteristic nucleotide base of DNA; X represents a deoxythymidine nucleotide (T) labelled with Atto 655 dye using conventional amine attachment chemistry and Q represents a deoxythymidine nucleotide labelled with a BHQ-2 quencher. It further comprises a capture region (A nucleotide) selective for capturing deoxythymidine triphosphate nucleotides (dTTPs).

Another single-stranded oligonucleotide B, comprising (1) a second oligonucleotide region having a sequence complementary to the 3' end of the first oligonucleotide with a single base mismatch; (2) a third oligonucleotide region having a sequence complementary to the 5' end of the first oligonucleotide and (3) a 76 base-pair single-stranded linker region, is also prepared. It has the following nucleotide sequence:
5'PCATGTTCGATGAGGCACGATAGATGTACGCTTTGACATACGCTTTGACAATACTTGAGCAGTCGGCAGA TATAGGATGTTGCAAGCTCCGTGAGTCCCACAAACCAAAAACCTCG3'
wherein additionally P represents a 5' phosphate group.

A reaction mixture comprising the probe system is then prepared having a composition corresponding to that derived from the following formulation:
56uL 5x buffer pH 7.5
28uL oligonucleotide A, 100 nM
28uL oligonucleotide B, 10 nM
2.8uL mixture of dNTPs (including dTTP), 10 nM
0.4U Phusion II Hot Start polymerase (exonuclease)
1.6U Bst Large Fragment DNA polymerase
20U *E. coli* ligase
4U Thermostable Inorganic Pyrophosphatase
Water to 280uL
wherein the 5x buffer comprises the following mixture:
   200uL Trizma hydrochloride, 1M, pH 7.5
   13.75uL aqueous MgCl₂, 1M
   2.5uL Dithiothreitol, 1M
   50uL Triton X-100 surfactant (10%)
   20uL Nicotinamide adenine dinucleotide, 100 uM
   166.67uL KCl
   Water to 1mL

In the presence of a single dTTP nucleotide, said nucleotide is incorporated onto the 3' end of one of the oligonucleotides B and ligation of oligonucleotide B to form a closed-loop used probe occurs. This process is carried out by incubating the mixture at 37°C for 50 minutes. The reaction medium is then incubated at 70°C for a further 50 minutes, activating the Phusion II polymerase. One of the oligonucleotides A can anneal to a circularised oligonucleotide B at this temperature and in this double-stranded form is digested by the polymerase, releasing its fluorophores into a detectable state. A further oligonucleotide B is then able to anneal to the circularised probe, allowing this process to repeat in a continuous cycle and resulting in a growth of fluorescence intensity over time.

Further sets of similar oligonucleotide probes are also prepared having different capture sites, sequences and fluorophores which, combined with the first probe set, allow the capture, detection and discrimination of the four nucleotide bases to be achieved.

SEQUENCE LISTING

<110>  Base4 Innovation Limited

<120>  DROPLET SEQUENCING DEVICE

<130>  P103087EP

<141>  2015-02-17

<160>  2

<170>  PatentIn version 3.5

<210>  1
<211>  40
<212>  DNA
<213>  Artificial sequence

<220>
<223>  oligonucleotide 1

<220>
<221>  misc_feature
<222>  (26)..(26)
<223>  deoxythymidine nucleotide (T) labelled with Atto 655 dye

<220>
<221>  misc_feature
<222>  (27)..(27)
<223>  deoxythymidine nucleotide (T) labelled with Atto 655 dye

<220>
<221>  misc_feature
<222>  (28)..(28)
<223>  deoxythymidine nucleotide labelled with a BHQ-2 quencher

<220>
<221>  misc_feature
<222>  (29)..(29)
<223>  deoxythymidine nucleotide (T) labelled with Atto 655 dye

<220>
<221>  misc_feature
<222>  (30)..(30)
<223>  deoxythymidine nucleotide (T) labelled with Atto 655 dye

<400>  1
tcgtgcctca tcgaacatga cgaggttttt ggtttgtggt                              40

<210>  2
<211>  115
<212>  DNA
<213>  Artificial sequence

<220>
<223>  oligonucleotide 2

<220>
<221>  misc_feature

<222>  (1)..(1)
<223>  5′ phosphate group attached to cytosine

<400>  2
catgttcgat gaggcacgat agatgtacgc tttgacatac gctttgacaa tacttgagca      60

gtcggcagat ataggatgtt gcaagctccg tgagtcccac aaaccaaaaa cctcg          115

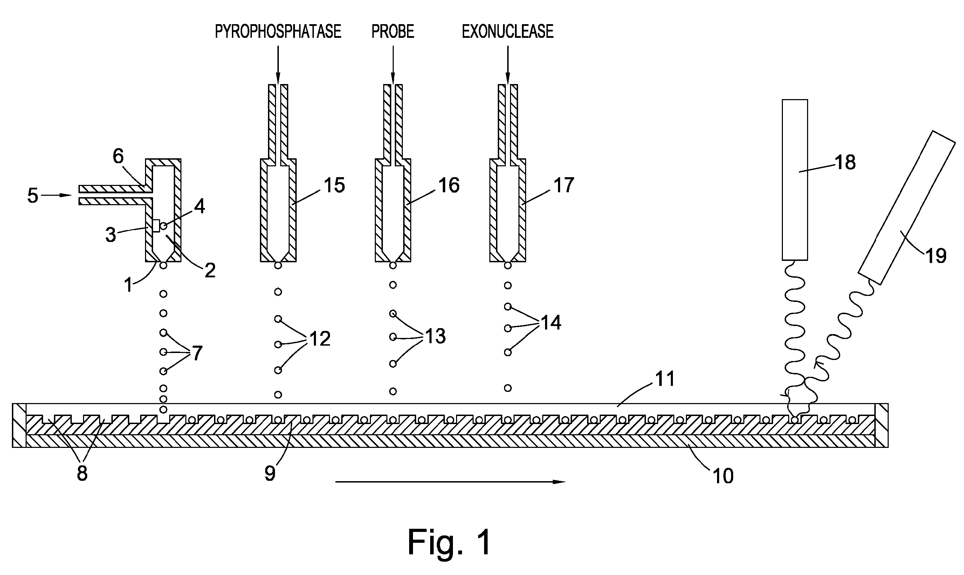

## Claims

1. An apparatus for sequencing a polynucleotide analyte comprising;
• a first zone in which a stream of single nucleotides is generated by progressive pyrophosphorolysis of a molecule of the analyte attached to a particle and exposed to a flowing aqueous medium;
• a second zone in which a corresponding stream of aqueous droplets is generated from the aqueous medium and the nucleotide stream and wherein at least some of the droplets contain a single nucleotide and
• a third zone in which each droplet is stored and/or interrogated to reveal a property characteristic of the single nucleotide it may contain;
**characterised in that** the first zone includes at least one chemically-modified substrate adapted to bind temporarily to a particle having at least two different regions adapted to bind respectively to the substrate and to the analyte.

2. An apparatus as claimed in claim 1 **characterised in that** the first zone comprises a microfluidic chamber through which the aqueous medium flows and the substrate is located within the chamber.

3. An apparatus as claimed in any of the preceding claims **characterised in that** the substrate is provided with first moieties adapted to bind to corresponding second moieties on the particle.

4. An apparatus as claimed in claim 3 **characterised in that** the first and second moieties are selected from the group of corresponding pairs consisting of avidin/biotin, streptavidin/biotin, polyhistidine/chelated metal ion, boronic acid/carbohydrate and maleimido/selenol.

5. An apparatus as claimed in claim 4 **characterised in that** the pair of first and second moieties comprises a first or second polyhistidine moiety comprised of a least six histidine residues and a second or first chelator moiety selected from a nitrotriacetate or iminodiacetate salt derivative of cobalt, copper or nickel.

6. An apparatus as claimed in any of the preceding claims **characterised in that** the droplets in the third zone contain at least one single-nucleotide probe selective for one of the nucleobase types from which the analyte is constituted; said probe(s) being capable of fluorescing substantially only after it has captured a single nucleotide and undergone subsequent exonucleolysis.

7. An apparatus as claimed in claim 6 **characterised in that** the probe comprises (a) a first single-stranded oligonucleotide labelled with characteristic fluorophores in an undetectable state and (b) second and third single-stranded oligonucleotides capable of hybridising to complementary regions on the first oligonucleotide.

8. An apparatus as claimed in claim 6 or claim 7 **characterised by** further comprising a means to introduce the probe(s) into the aqueous medium before, as or after each droplet is created.

9. An apparatus as claimed in any of the preceding claims **characterised in that** the second zone includes a printer nozzle adapted to print each droplet into a third zone including a surface comprised of an array of droplet-receiving locations.

10. An apparatus as claimed in claim 9 **characterised in that** the droplet-receiving locations are moveable relative to the printer nozzle.

11. An apparatus as claimed in claim 9 or claim 10 **characterised in that** the volume of each droplet-receiving location is a well having a volume greater than that of the droplet printed thereinto thereby enabling further reactant-containing droplets to be added either after or beforehand.

12. An apparatus as claimed in any of the preceding claims **characterised in that** the third zone includes an interrogation means for detecting fluorescence radiation emitted from each droplet.

13. An apparatus as claimed in claim 12 **characterised by** further comprising a microprocessor for sequencing the analyte by analysing data generated by the interrogation means.

14. An apparatus for sequencing a polynucleotide analyte **characterised by** comprising:
• at least one microfluidic chamber containing a substrate chemically-modified to bind reversibly to a complementary particle bearing a molecule of the analyte;
• a means for delivering and retrieving the particle to and from the chamber;
• microfluidic channels for passing an aqueous pyrophosphorolysing medium through the microfluidic chamber and across the substrate;
• at least one first printer nozzle adapted to receive a nucleotide-containing aqueous medium from the microfluidic chamber and to create microdroplets therefrom;
• at least one sheet moveable relative to the first printer nozzle patterned with an array of wells for receiving the nucleotide-containing microdroplets from the printer nozzle(s);
• at least one additional printer nozzle type moveable relative to the sheet each additional type being adapted to deliver a microdroplet containing a different reagent into the wells either before or after a nucleotide-containing microdroplet has been received;
• a means for moving the first and additional printer nozzle types relative to the sheet and
• a means for coating the sheet with a liquid immiscible with the nucleotide-containing medium.

15. An apparatus as claimed in claim 14 **characterised by** further comprising at least one laser for interrogating the contents of the wells and at least one photodetector for detecting electromagnetic radiation originating therefrom each moveable relative to the sheet.

16. A method of pyrophosphorolysing a polynucleotide **characterised by** the steps of (1) binding the polynucleotide to a particle the surface of which is adapted to (a) bind the polynucleotide and (b) bind a substrate surface reversibly; (2) binding the particle to the substrate surface; (3) performing pyrophosphorolysis on the polynucleotide bound to the particle in a flowing medium and (4) releasing the particle from the surface.
